(19) Europäisches Patentamt
European Patent Office
Office européen des brevets

(11) Veröffentlichungsnummer : **0 086 404**
**B1**

(12) # EUROPÄISCHE PATENTSCHRIFT

(45) Veröffentlichungstag der Patentschrift : **30.12.86**

(21) Anmeldenummer : **83101008.7**

(22) Anmeldetag : **03.02.83**

(51) Int. Cl.⁴ : **C 07 C177/00**, C 07 C 59/46, A 61 K 31/19, A 61 K 31/557// C07D317/72, C07F9/40

(54) **Neue Carbacycline, Verfahren zu ihrer Herstellung und ihre Verwendung als Arzneimittel.**

(30) Priorität : **08.02.82 DE 3204443**

(43) Veröffentlichungstag der Anmeldung : **24.08.83 Patentblatt 83/34**

(45) Bekanntmachung des Hinweises auf die Patenterteilung : **30.12.86 Patentblatt 86/52**

(84) Benannte Vertragsstaaten : **AT BE CH DE FR GB IT LI LU NL SE**

(56) Entgegenhaltungen :
**EP-A- 0 011 591**
**ANGEWANDTE CHEMIE, International Edition, Band 20, Nr. 12, Dezember 1981, Seiten 1046-1048, Verlag Chemie GmbH, Weinheim, DE. W. SKUBALLA et al.: "A new route to 6a-carbacyclins - Synthesis of a stable, biologically potent prostacyclin analogue"**
**CHEMICAL ABSTRACTS, Band 95, Nr. 11, 14. September 1981, Seite 86, Nr. 91359z, Columbus, Ohio, USA R. CESARANI et al.: "5E-13,14-didehydro-carboprostacyclin and 5E-13,14-didehydro-20-methyl-carboprostacyclin: preliminary data on inhibition of platelet aggregation and hypotensive effect"**
**Die Akte enthält technische Angaben, die nach dem Eingang der Anmeldung eingereicht wurden und die nicht in dieser Patentschrift enthalten sind.**

(73) Patentinhaber : **SCHERING AKTIENGESELLSCHAFT**
**Berlin und Bergkamen**
**Müllerstrasse 170/178 Postfach 65 03 11**
**D-1000 Berlin 65 (DE)**

(72) Erfinder : **Skuballa, Werner, Dr.**
**Olwenstrasse 25**
**D-1000 Berlin 28 (DE)**
Erfinder : **Radüchel, Bernd, Dr.**
**Gollanczstrasse 132**
**D-1000 Berlin 28 (DE)**
Erfinder : **Vorbrüggen, Helmut, Prof.**
**Wilkestrasse 7**
**D-1000 Berlin 27 (DE)**
Erfinder : **Stenzel, Jorge Casals, Dr.**
**Sertoriusring 295**
**D-6500 Mainz (DE)**
Erfinder : **Mannesmann, Gerda, Dr.**
**Sachsenring 22-24**
**D-5000 Köln (DE)**
Erfinder : **Town, Michael Harold, Dr.**
**Buchsbaumweg 3**
**D-1000 Berlin 47 (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 086 404 B1

Jouve, 18, rue St-Denis, 75001 Paris, France

**Beschreibung**

Die vorliegende Erfindung betrifft (5E)-13,14,18,18,19,19-Hexadehydro-6a-carba-prostaglandin-$I_2$-derivate, deren physiologisch verträgliche Salze, Verfahren zu ihrer Herstellung und diese enthaltende pharmazeutische Zusammensetzungen.

In der deutschen Offenlegungsschrift 28 45 770 werden Carbacyclinderivate der allgemeinen Formel

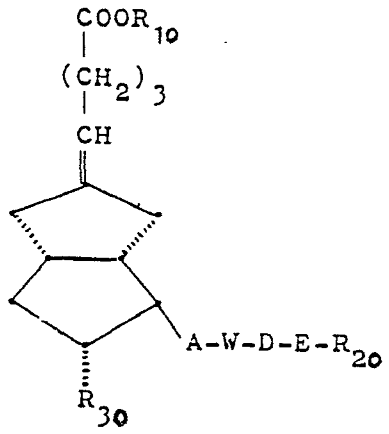

beansprucht, in der

$R_{10}$ Wasserstoff, Alkyl, Cycloalkyl, Aryl oder einen heterocyclischen Rest bedeuten kann,

A eine $—CH_2—CH_2$-, trans —CH=CH- oder —C≡C-Gruppe,

W eine freie oder funktionell abgewandelte Hydroxymethylengruppe oder eine freie oder funktionell abgewandelte

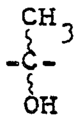

Gruppe, wobei die OH-Gruppe α-ständig ist,

D und E gemeinsam eine direkte Bindung oder

D eine geradkettige oder verzweigte gesättigte oder ungesättigte Alkylengruppe mit 1-10 C-Atomen, die gegebenenfalls durch Fluoratome substituiert ist,

E eine Sauerstoffatom oder eine —C≡C-Bindung oder eine direkte Bindung,

$R_{20}$ eine Alkyl-, Cycloalkyl-, oder eine gegebenenfalls substituierte Aryl- oder eine heterocyclische Gruppe,

$R_{30}$ eine freie oder funktionell abgewandelte Hydroxygruppe, und falls $R_{10}$ die Bedeutung eines Wasserstoffatoms hat, deren Salze mit physiologisch verträglichen Basen bedeuten.

Die Verbindungen besitzen die für Prostacycline typischen Eigenschaften, wie z. B. Senkung des peripheren arteriellen und koronaren vaskulären Widerstandes, Inhibierung der Thrombozytenaggregation und Auflösung von Plättchenthromben, myocardiale Zytoprotektion und damit Senkung des systemischen Blutdrucks, ohne zugleich Schlagvolumen und koronare Durchblutung zu senken, Behandlung von Schlaganfall, Prophylaxe und Therapie koronarer Herzerkrankungen, koronarer Thrombose, des Herzinfarkts, peripherer Arterienerkrankungen, Arteriosklerose und Thrombose, Therapie des Schocks, Inhibierung der Bronchokonstriktion, Inhibierung der Magensäuresekretion, Zytoprotektion der Magen- und Darmschleimhaut, antiallergische Eigenschaften, Senkung des pulmonaren vaskulären Widerstandes und des pulmonaren Blutdrucks, Förderung der Nierendurchblutung, Anwendung anstelle von Heparin oder als Adjuvans bei der Dialyse der Hämofiltration, Konservierung von Blutplasmakonserven, besonders von Blutplättchenkonserven, Inhibierung von Geburtswehen, Behandlung von Schwangerschaftstoxikose, Erhöhung der zerebralen Durchblutung etc. Außerdem besitzen die neuen Prostaglandinanaloga antiproliferative Eigenschaften.

In Angew. Chem. Int. Ed. Engl. 20 (1981), 1046 werden Carbacycline mit einer 13,14-Doppelbindung beschrieben, die teilweise in 18-Stellung dreifach ungesättigt sind. In Chem. Abstr. 95 (1981), 91359z werden Carbacycline mit einer 13,14-Dreifachbindung erwähnt.

Unter den in der DE-OS 28 45 770 beanspruchten Verbindungen zeigten die (5E)-13,14,18,18,19,19-Hexadehydro-6a-carbaprostaglandin-$I_2$-Verbindungen als blutdrucksenkende Mittel und thrombozytenaggregationshemmende Mittel derartig herausragende Eigenschaften, daß damit die Dosierung weiter herabgesetzt werden kann, wodurch auch unerwünschte Nebenwirkungen noch stärker zurückgedrängt werden. Die (5E)-13,14,18,18,19,19-Hexadehydro-6a-carba-prostaglandin-$I_2$-Verbindungen sind in der

DE-OS 28 45 770 nicht namentlich beschrieben. Verbindungen mit A als —C≡C-Gruppe werden gegenüber den anderen Verbindungen, in denen A eine $-CH_2-CH_2$- oder trans-CH=CH-Gruppe bedeutet, nicht herausgestellt.

Die Nomenklatur der Verbindungen basiert auf einem Vorschlag von Morton und Brokaw (J. Org. Chem. *44*, 2280 [1979]). (5E)-6a-Carba-prostaglandin-$I_2$ hat danach folgende Strukturformel :

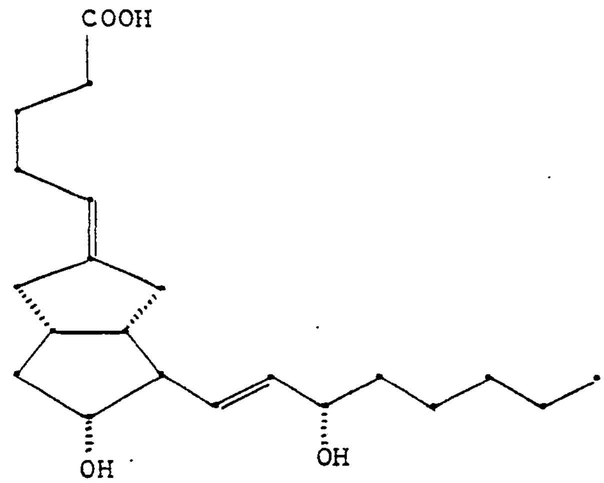

Die Erfindung betrifft somit (5E)-13,14,18,18,19,19-Hexadehydro-6a-carba-prostaglandin-$I_2$-Derivate der allgemeinen Formel I

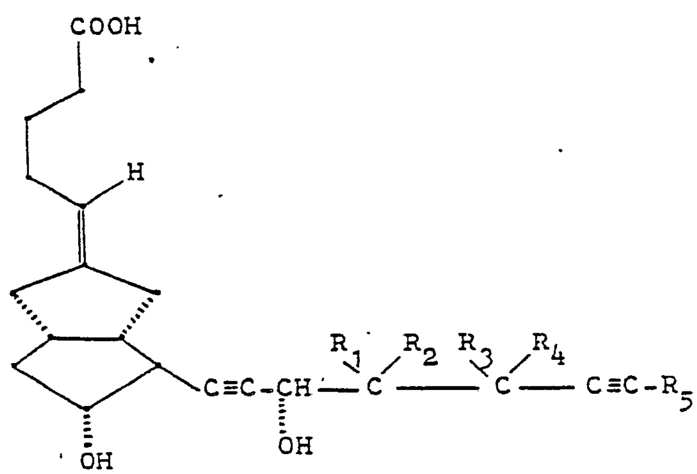

(I)

worin

$R_1$, $R_2$, $R_3$, $R_4$ ein Wasserstoffatom oder eine Alkylgruppe mit 1-2 C-Atomen,

$R_5$ eine Alkylgruppe mit 1-2 C-Atomen bedeuten, sowie deren Salze mit physiologisch verträglichen Basen.

Als Alkylgruppen $R_1$, $R_2$, $R_3$, $R_4$, $R_5$ kommen Methyl und Äthyl in Betracht.

Die Erfindung betrifft auch (5E)-(16RS)-20-Äthyl-13,14-didehydro-16-methyl-18,18,19,19-tetradehydro-6a-carba-prostaglandin-$I_2$.

Zur Salzbildung sind anorganische und organische Basen geeignet, wie sie dem Fachmann zur Bildung physiologisch verträglicher Salze bekannt sind. Beispielsweise genannt seien Alkalihydroxide, wie Natrium- und Kaliumhydroxid, Erdalkalihydroxid wie Calciumhydroxid, Ammoniak, Amine, wie Äthanolamin, Diäthanolamin, Triäthanolamin, N-Methylglucamin, Morpholin, Tris-(hydroxymethyl)-methylamin usw.

Die Erfindung betrifft ferner ein Verfahren zur Herstellung der erfindungsgemäßen Prostacyclinderivate der allgemeinen Formel I, dadurch gekennzeichnet, daß man eine Verbindung der allgemeinen Formel II

(Siehe Figur Seite 4 f.)

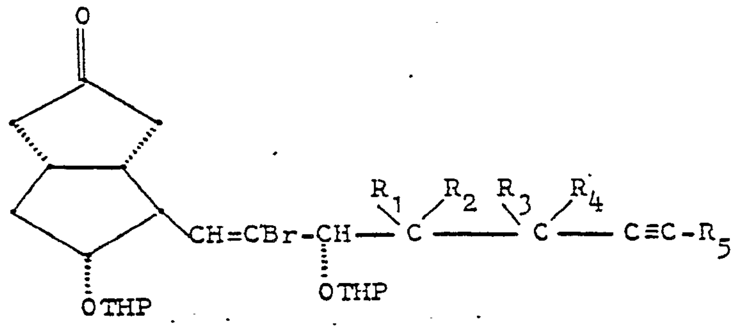

(II)

worin $R_1$, $R_2$, $R_3$, $R_4$, $R_5$ die oben angegebenen Bedeutungen aufweisen und THP den Tetrahydropyranylrest bedeutet mit einem Wittig-Reagenz der Formel III

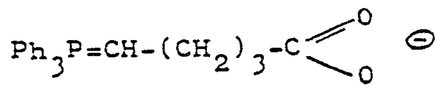

(III)

worin Ph eine Phenylgruppe bedeutet, unter gleichzeitiger HBr-Abspaltung umsetzt und anschließend Isomere trennt, Schutzgruppen abspaltet und gegebenenfalls die Carboxylgruppe mit einer physiologisch verträglichen Base in ein Salz überführt.

Die Umsetzung der Verbindung der allgemeinen Formel II mit dem Wittig-Reagenz der Formel III, das man aus dem entsprechenden Phosphoniumsalz mit Methansulfinylmethylnatrium oder Methansulfinylmethylkalium oder Kalium-tert.-butylat in Dimethylsulfoxid oder Dimethylsulfoxid-Tetrahydrofurangemischen herstellt, wird bei Temperaturen von 0 °C bis 100 °C, vorzugsweise 20 °C bis 60 °C, in einem aprotischen Lösungsmittel oder Lösungsmittelgemisch, vorzugsweise Dimethylsulfoxid, Dimethylformamid oder Tetrahydrofuran, vorgenommen. Die Trennung der dabei erhaltenen Z- und E-konfigurierten Olefine erfolgt auf übliche Art, beispielsweise durch Säulen- oder Schichtchromatographie. Bei der vorstehend beschriebenen Wittig-Olefinierung erfolgt gleichzeitig unter Abspaltung von Bromwasserstoff die Bildung der 13,14-Acetylenbindung.

Die Schutzgruppenabspaltung wird in einer wäßrigen Lösung einer organischen Säure, wie zum Beispiel Essigsäure, Propionsäure u. a. oder in einer wäßrigen Lösung einer anorganischen Säure, wie zum Beispiel Salzsäure, durchgeführt. Zur Verbesserung des Löslichkeit wird zweckmäßigerweise ein mit Wasser mischbares inertes organisches Lösungsmittel zugesetzt. Geeignete organische Lösungsmittel sind zum Beispiel Alkohole, wie Methanol und Äthanol, und Äther, wie Dimethoxyäthan, Dioxan und Tetrahydrofuran. Tetrahydrofuran wird bevorzugt angewendet. Die Abspaltung wird vorzugsweise bei Temperaturen zwischen 20 °C und 30 °C durchgeführt.

Die Carbonsäuren der allgemeinen Formel I können mit geeigneten Mengen der entsprechenden anorganischen Basen unter Neutralisierung in Salze überführt werden. Beispielsweise erhält man beim Lösen der entsprechenden Säuren in Wasser, welches die stöchiometrische Menge Base enthält, nach Abdampfen des Wassers oder nach Zugabe eines mit Wasser mischbaren Lösungsmittels, zum Beispiel Alkohol oder Aceton, das feste anorganische Salz. Zur Herstellung eines Aminsalzes wird die PG-Säure in einem geeigneten Lösungsmittel, beispielsweise Äthanol, Aceton, Diäthyläther oder Benzol gelöst und mindestens die stöchiometrische Menge des Amins dieser Lösung zugesetzt. Dabei fällt das Salz gewöhnlich in fester Form an oder wird nach Verdampfen des Lösungsmittels in üblicher Weise isoliert.

Die als Ausgangsmaterial dienenden Verbindungen der allgemeinen Formel II können beispielsweise hergestellt werden, indem man in an sich bekannter Weise einen Aldehyd der Formel IV (DE-OS 28 45 770).

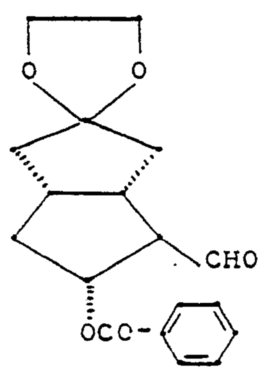

(IV)

mit einem Phosphonat der allgemeinen Formel V

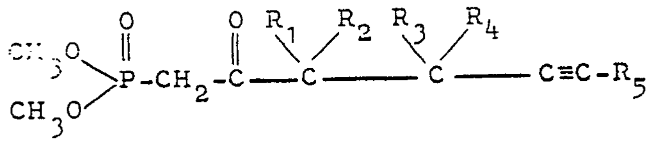

(V)

worin $R_1$, $R_2$, $R_3$, $R_4$ und $R_5$ die oben angegebenen Bedeutungen aufweisen, in Gegenwart eines Deprotonierungsmittels, wie beispielsweise Natriumhydrid oder Kalium-tert.-butylat und eines Bromierungsmittels, wie beispielsweise N-Bromsuccinimid, zu einem Keton der allgemeinen Formel VI umsetzt.

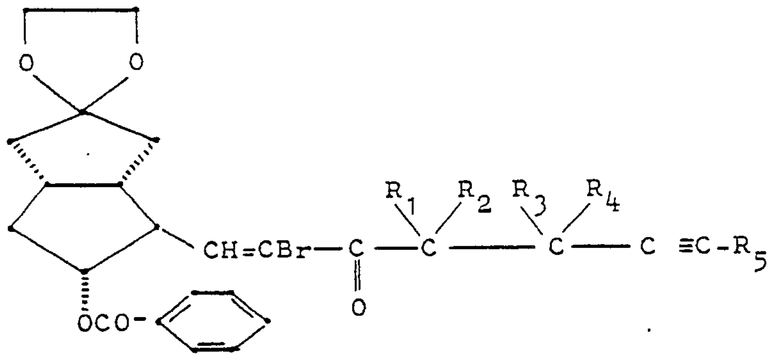

(VI)

Nach Reduktion der Ketogruppe mit Natriumborhydrid und gegebenenfalls Epimerentrennung, Verseifung der Estergruppe beispielsweise mit Kaliumcarbonat in Methanol und Ketalspaltung mit wäßriger Essigsäure sowie gegebenenfalls Epimerentrennung gelangt man zum Keton der allgemeinen Formel VII

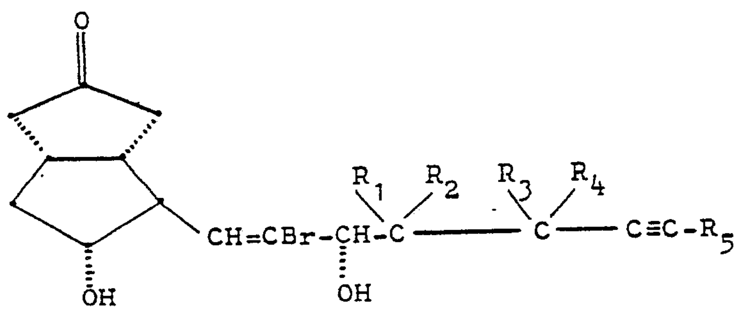

(VII)

Die Verätherung der Hydroxylgruppen mit Dihydropyran in Gegenwart katalytischer Mengen p-Toluolsulfonsäure liefert die Verbindungen der allgemeinen Formel II.

Die Herstellung der Phosphonate der allgemeinen Formel V erfolgt in an sich bekannter Weise durch Umsetzung eines Alkylhalogenids (herstellbar aus dem entsprechenden Alkohol durch Halogenierung) der allgemeinen Formel VIII

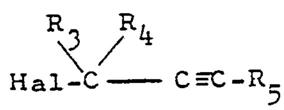

(VIII)

mit dem aus dem Phosphonat der allgemeinen Formel IX erzeugten Dianions,

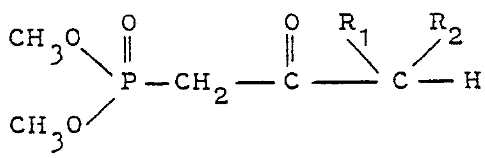

(IX)

worin $R_1$, $R_2$, $R_3$, $R_4$ und $R_5$ die oben angegebenen Bedeutungen aufweisen.

Einen weiteren Zugang zu den Phosphonaten der allgemeinen Formel V besteht in der Umsetztung des Anions von Methylphosphonsäuredimethylester mit einem Ester der allgemeinen Formel X

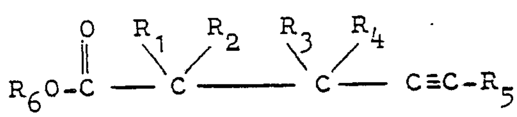

(X)

worin $R_1$, $R_2$, $R_3$, $R_4$ und $R_5$ die oben angegebenen Bedeutungen aufweisen und $R_6$ eine Alkylgruppe mit 1-5 C-Atomen bedeutet, den man aus dem entsprechenden Malonsäureester durch Alkylierung mit dem

Halogenid der allgemeinen Formel VIII und anschließender Decarbalkoxylierung erhalten kann. Der Ester der allgemeinen Formel X ist auch aus der Carbonsäure der allgemeinen Formel XI

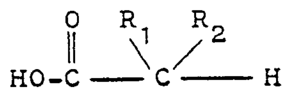

(XI)

worin $R_1$ und $R_2$ die oben angegebenen Bedeutungen aufweisen durch Alkylierung mit dem Halogenid der allgemeinen Formel VIII und anschließender Veresterung zugänglich.

Die Verbindungen dieser Erfindung wirken blutdrucksenkend und bronchodilatorisch. Sie sind weiterhin geeignet zur Hemmung der Thrombozyten-Aggregation. Folglich stellen die neuen Prostacyclin-Derivate der Formel I wertvolle pharmazeutische Wirkstoffe dar. Darüber hinaus weisen sie bei ähnlichem Wirkungsspektrum, verglichen mit entsprechenden Prostaglandinen, eine höhere Spezifität und vor allem eine wesentlich längere Wirksamkeit auf. Im Vergleich zu $PGI_2$ zeichnen sie sich durch größere Stabilität aus. Die hohe Gewebsspezifität der neuen Prostaglandine zeigt sich bei der Untersuchung an glattmuskulären Organen, wie z. B. am Meerschweinchenileum oder an der isolierten Kaninchentrachea, wo eine wesentlich geringere Stimulation zu beobachten ist als bei der Applikation natürlicher Prostaglandine vom E-, A- oder F-Typ.

Die neuen Carbacyclin-Derivate besitzen die für Prostacycline typischen Eigenschaften, wie z. B. Senkung des peripheren arteriellen und koronaren vaskulären Widerstandes, Inhibierung der Thrombozytenaggregation und Auflösung von Plättchenthromben, myocardiale Zytoprotektion und damit Senkung des systemischen Blutdrucks ohne zugleich Schlagvolumen und koronare Durchblutung zu senken ; Behandlung von Schlaganfall, Prophylaxe und Therapie koronarer Herzerkrankungen, koronarer Thrombose, des Herzinfarkts, peripherer Arterienerkrankungen, Arteriosklerose und Thrombose, Prophylaxe und Therapie ischaemischer Attacken des ZNS-Systems, Therapie des Schocks, Inhibierung der Bronchokonstriktion, Inhibierung der Magensäuresekretion, Zytoprotektion der Magen- und Darmschleimhaut, Zytoprotektion in der Leber und im Pankreas ; antiallergische Eigenschaften, Senkung des pulmonaren vaskulären Widerstandes und des pulmonaren Blutdrucks, Förderung der Nierendurchblutung, Anwendung anstelle von Heparin oder als Adjuvans bei der Dialyse der Hämofiltration, Konservierung von Blutplasmakonserven, besonders von Blutplättchenkonserven, Inhibierung von Geburtswehen, Behandlung von Schwangerschaftstoxikose, Erhöhung der zerebralen Durchblutung etc. Außerdem besitzen die neuen Carbacyclin-Derivate antiproliferative Eigenschaften. Die Carbacycline dieser Erfindung können auch in Kombination, z. B. mit β-Blockern oder Diuretika, verwendet werden.

Die Dosis der Verbindungen ist 1-1 500 μg/kg/Tag, wenn sie am menschlichen Patienten verabreicht werden. Die Einheitsdosis für den pharmazeutischen akzeptablen Träger beträgt 0,01-100 mg.

Bei intravenöser Injektion an wachen, hypertonen Ratten in Dosen von 5, 20 und 100 μg/kg Körpergewicht zeigen die erfindungsgemäßen Verbindungen eine stärkere blutdrucksenkende und länger anhaltende Wirkung als $PGE_2$ und $PGA_2$, ohne wie $PGE_2$ Durchfälle oder $PGA_2$ kardiale Arrhythmien auszulösen.

Bei intravenöser Injektion an narkotisierten Kaninchen zeigen die erfindungsgemäßen Verbindungen im Vergleich zu $PGE_2$ und $PGA_2$ eine stärkere und erheblich länger anhaltende Blutdrucksenkung, ohne daß andere glattmuskuläre Organe oder Organfunktionen beeinflußt werden.

Für die parenterale Verabreichung werden sterile, injizierbare, wäßrige oder ölige Lösungen benutzt. Für die orale Applikation sind beispielsweise Tabletten, Dragées oder Kapseln geeignet.

Die Erfindung betrifft damit auch Arzneimittel auf Basis der Verbindungen der allgemeinen Formel I und üblicher Hilfs- und Trägerstoffe.

Die erfindungsgemäßen Wirkstoffe sollen in Verbindung mit den in der Galenik bekannten und üblichen Hilfsstoffen z. B. zur Herstellung von Blutdrucksenkern dienen.

## Beispiel 1

(5E)-(16RS)-13,14-Didehydro-16-methyl-18,18,19,19-tetradehydro-6a-carba-prostaglandin-$I_2$

Zu einer Lösung von 9,4 g 4-Carboxybutyltriphenylphosphoniumbromid in 20 ml Dimethylsulfoxid und 7,8 ml Tetrahydrofuran fügt man bei 5 °C innerhalb von 45 Minuten 4,75 g Kalium-tert.-butylat und rührt 45 Minuten bei 5 °C nach. Zur roten Ylenlösung fügt man eine Lösung von 1,83 g (1R,5S,6R,7R)-7-(tetrahydropyran-2-yloxy)-6-[(3S,4RS)-2-brom-4-methyl-3-(tetrahydropyran-2-yloxy)-oct-1-en-6-inyl]-bicyclo[3.3.0]octan-3-on in 3 ml Tetrahydrofuran und rührt 4 Stunden bei 40 °C. Das Reaktionsgemisch wird auf Eiswasser gegossen, mit 35 %iger Zitronensäurelösung auf pH 4-5 angesäuert und dreimal mit Methylenchlorid extrahiert. Die organische Phase wird mit Sole geschüttelt, über Magnesiumsulfat getrocknet und im Vakuum eingedampft. Den Rückstand reinigt man durch Chromatographie an Kieselgel. Mit Hexan/Essigester (3 + 2) erhält man zunächst 180 mg des 5Z-konfigurierten Olefins sowie als polarere Komponente 680 mg (5E)-(16RS)-13,14-Didehydro-16-methyl-18,18,19,19-tetradehydro-6a-carba-prostaglandin-$I_2$-11,15-bis-(tetrahydro-pyranyläther) als farbloses Öl.

IR ($CHCl_3$) : 3 500 (breit), 2 940, 2 860, 2 225, 1 710, 1 440/cm.

Zur Schutzgruppenabspaltung rührt man 680 mg des vorstehend erhaltenen Olefinierungsprodukts mit 25 ml einer Mischung aus Essigsäure/Wasser/Tetrahydrofuran (65/35/10) 20 Stunden bei 25 °C. Anschließend dampft man im Vakuum ein und chromatographiert den Rückstand an Kieselgel. Mit Essigester/Essigsäure (99,9 + 0,1) erhält man 345 mg der Titelverbindung als farbloses Öl.

IR : 3 600, 3 400 (breit), 2 930, 2 225, 1 710, 1 603, 1 020/cm.

Das Ausgangsmaterial für die obige Titelverbindung wird wie folgt hergestellt :

a) (1R,5S,6R,7R)-3,3-Äthylendioxy-7-benzoyloxy-6-[(4RS)-2-brom-4-methyl-3-oxo-oct-1-en-6-inyl]-bicyclo[3.3.0]octan

Zu einer Suspension von 1,81 g Natriumhydrid in 180 ml Dimethoxyäthan tropft man bei 0 °C eine Lösung von 10,5 g 3-Methyl-2-oxo-hept-5-in-phosphonsäuredimethylester in 70 ml Dimethoxyäthan, rührt 1 Stunde bei 0 °C und fügt dann 7,4 g fein gepulvertes N-Bromsuccinimid hinzu. Man rührt 30 Minuten bei 0 °C, versetzt mit einer Lösung aus 11,4 g (1R,5S,6R,7R)-3,3-Äthylendioxy-7-benzoyloxy-6-formyl-bicyclo[3.3.0]-octan in 90 ml Dimethoxyäthan und rührt 2 Stunden bei 0 °C. Das Reaktionsgemisch gießt man auf gesättigte Ammoniumchloridlösung und extrahiert mit Äther. Man wäscht den organischen Extrakt mit Wasser neutral, trocknet über Magnesiumsulfat und dampft im Vakuum ein. Nach Chromatographie des Rückstands an Kieselgel erhält man mit Hexan/Äther (3 + 2) 8,2 g des ungesättigten Ketons als farbloses Öl.

IR : 2 930, 2 880, 1 712, 1 688, 1 602, 1 595, 1 450, 1 275, 945/cm.

b) (1R,5S,6R,7R)-7-Hydroxy-6-[(3S,4RS)-2-brom-3-hydroxy-4-methyl-oct-1-en-6-inyl]-bicyclo[3.3.0]octan-3-on.

Zu einer Lösung von 5,9 g des nach Beispiel 1a) hergestellten Ketons in 140 ml Methanol fügt man bei — 40 °C portionsweise 2,5 g Natriumborhydrid und rührt 30 Minuten bei — 40 °C. Anschließend verdünnt man mit Äther, wäscht mit Wasser neutral, trocknet über Magnesiumsulfat und dampft im Vakuum ein.

Das Rohprodukt (15-Epimerengemisch) löst man in 200 ml Methanol, fügt 2,5 g Kaliumcarbonat zu und rührt 17 Stunden bei 23 °C unter Argon. Anschließend engt man im Vakuum ein, verdünnt mit Äther und wäscht mit Sole neutral. Man trocknet über Magnesiumsulfat und dampft im Vakuum ein.

Den Eindampfrückstand rührt man 16 Stunden bei Raumtemperatur mit 300 ml einer Mischung aus Essigsäure/Wasser/Tetrahydrofuran (65/35/10) und dampft anschließend im Vakuum ein. Durch Säulenchromatographie an Kieselgel mit Äther/Methylenchlorid erhält man zunächst 1,6 g des 15β-konfigurierten Alkohols sowie als polarere Komponente 2,1 g der Titelverbindung (PG-Nomenklatur 15α-Hydroxy) als farblose Öle.

IR : 3 600, 3 430 (breit), 2 960, 2 920, 2 870, 1 738, 1 600, 1 400/cm.

c) (1R,5S,6R,7R)-7-(tetrahydropyran-2-yloxy)-6-[(3S,4RS)-2-brom-4-methyl-3-(tetrahydropyran-2-yloxy)-oct-1-en-6-inyl]-bicyclo[3.3.0]octan-3-on

Eine Lösung aus 1,6 g des nach Beispiel 1b) hergestellten α-Alkohols, 16 mg p-Toluolsulfonsäure und 1,5 g Dihydropyran in 50 ml Methylenchlorid rührt man 35 Minuten bei 0 °C. Anschließend verdünnt man mit Äther, schüttelt mit verdünnter Natriumbicarbonat-Lösung, wäscht mit Wasser neutral, trocknet über Magnesiumsulfat und dampft im Vakuum ein. Nach Chromatographie des Rückstands an Kieselgel erhält man mit Hexan/Äther (7 + 3) 2,17 g der Titelverbindung als farbloses Öl.

IR : 2 940, 2 870, 1 735, 1 450, 1 120, 1 018, 965/cm.

Beispiel 2

(5E)-(16RS)-13,14-Didehydro-16,20-dimethyl-18,18,19,19-tetradehydro-6a-carba-prostaglandin-$I_2$

In Analogie zu Beispiel 1 erhält man aus 1,6 g (1R,5S,6R,7R)-7-(Tetrahydropyran-2-yloxy)-6-[(3S,4RS)-2-brom-4-methyl-3-(tetrahydropyran-2-yloxy)-non-1-en-6-inyl]-bicyclo[3.3.0]octan-3-on 640 mg (5E)-(16RS)-13,14-Didehydro-16,20-dimethyl-18,18,19,19-tetradehydro-6a-carba-prostaglandin-$I_2$-11,15-bis-(tetrahydropyranyläther) als farbloses Öl.

IR : 3 500 (breit), 2 942, 2 860, 2 224, 1 710/cm.

Nach Abspaltung der Schutzgruppen gemäß Beispiel 1 erhält man 0,3 g der Titelverbindung als farbloses Öl.

IR : 3 600, 3 350 (breit), 2 932, 2 224, 1 710, 1 602/cm.

Das Ausgangsmaterial für die obige Titelverbindung wird wie folgt hergestellt :

a) (1R,5S,6R,7R)-3,3-Äthylendioxy-7-benzoyloxy-6-[(4RS,2-brom-4-methyl-3-oxo-non-1-en-6-inyl]-bicyclo[3.3.0]octan

In Analogie zu Beispiel 1a) erhält man aus 6 g 3-Methyl-2-oxo-oct-5-inyl-phosphonsäure-dimethy-

lester 3,7 g N-Bromsuccinimid und 5,6 g (1R,5S,6R,7R)-3,3-Äthylendioxy-7-benzoyloxy-6-formyl-bicyclo[3.3.0]octan 4,0 g des ungesättigten Ketons als farbloses Öl.
IR : 2 935, 2 883, 1 713, 1 687, 1 602, 1 596, 1 275, 947/cm.

b) (1R,5S,6R,7R)-7-Hydroxy-6-[(3S,4RS)-2-brom-3-hydroxy-4-methyl-non-1-en-6-inyl]-bicyclo[3.3.0]octan-3-on

In Analogie zu Beispiel 1b) erhält man aus 3 g des nach Beispiel 2a) hergestellten Ketons nach Reduktion mit 1,3 g Natriumborhydrid, Verseifung mit 1,2 g Kaliumkarbonat und anschließender Ketalspaltung mit 150 ml einer Mischung aus Essigsäure/Wasser/Tetrahydrofuran 1,2 g der Titelverbindung (15α-Hydroxy) als farbloses Öl.
IR : 3 610, 3 400 (breit), 2 960, 2 870, 1 739, 1 600/cm.

c) (1R,5S,6R,7R)-7-(Tetrahydropyran-2-yloxy)-6-[(3S,4RS)-2-brom-4-methyl-3-(tetrahydropyran-2-yloxy)-non-1-en-6-inyl]-bicyclo[3.3.0]octan-3-on

In Analogie zu Beispiel 1c) erhält man aus 0,78 g des nach Beispiel 2b) hergestellten Diols und 0,7 g Dihydropyran 1,1 g der Titelverbindung als farbloses Öl.
IR : 2 940, 2 872, 1 736, 1 450, 1 120, 965/cm.

## Beispiel 3

(5E)-(16RS)-20-Äthyl-13,14-didehydro-16-methyl-18,18,19,19-tetradehydro-6a-carba-prostaglandin-$I_2$

In Analogie zu Beispiel 1 erhält man aus 2 g (1R,5S,6R,7R)-7-(Tetrahydropyran-2-yloxy)-6-[(3S,4RS)-2-brom-4-methyl-3-(tetrahydropyran-2-yloxy)-dec-1-en-6-inyl]-bicyclo[3.3.0]octan-3-on 900 mg (5E)-(16RS)-20-Äthyl-13,14-didehydro-16-methyl-18,18,19,19-tetradehydro-6a-carba-prostaglandin-$I_2$-11,15-bis-(tetrahydropyranyläther) als farbloses Öl.
IR : 3 500 (breit), 2 948, 2 862, 2 220, 1 708/cm.
Nach Abspaltung der Schutzgruppen gemäß Beispiel 1 erhält man 420 mg der Titelverbindung als farbloses Öl.
IR : 3 600, 3 360 (breit), 2 930, 2 858, 2 220, 1 708, 1 601/cm.
Das Ausgangsmaterial für die obige Titelverbindung wird wie folgt hergestellt :

a) (1R,5S,6R,7R)-3,3-Äthylendioxy-7-benzoyloxy-6-[(4RS)-2-brom-4-methyl-3-oxo-dec-1-en-6-inyl]bicyclo[3.3.0]octan

In Analogie zu Beispiel 1a) erhält man aus 6,25 g 3-Methyl-2-oxo-non-5-inyl-phosphonsäure-dimethylester, 3,5 g N-Bromsuccinimid und 5,6 g (1R,5S,6R,7R)-3,3-Äthylendioxy-7-benzoyloxy-6-formyl-bicyclo[3.3.0]octan 4,5 g des ungesättigten Ketons als farbloses Öl.
IR : 2 940, 2 880, 1 712, 1 688, 1 601, 1 592, 1 275, 948/cm.

b) (1R,5S,6R,7R)-7-Hydroxy-6-[(3S,4RS)-2-brom-3-hydroxy-4-methyl-dec-1-en-6-inyl]-bicyclo[3.3.0]octan-3-on

In Analogie zu Beispiel 1b) erhält man aus 4 g der nach Beispiel 3a) hergestellten Ketons nach Reduktion mit Natriumborhydrid, Verseifung mit Kaliumcarbonat und anschließender Ketalspaltung mit Essigsäure/Wasser/Tetrahydrofuran (65/35/10) 1,5 g der Titelverbindung (15α-Hydroxy) als farbloses Öl.
IR : 3 610, 3 400 (breit), 2 955, 2 868, 1 738, 1 601/cm.

c) (1R,5S,6R,7R)-7-(Tetrahydropyran-2-yloxy-6-[(3S,4RS)-2-brom-4-methyl-3-(tetrahydropyran-2-yloxy)-dec-1-en-6-inyl]-bicyclo[3.3.0]octan-3-on

In Analogie zu Beispiel 1c) erhält man aus 1,2 g des nach Beispiel 3b) hergestellten Diols 1,81 g der Titelverbindung als farbloses Öl.
IR : 2 942, 2 868, 1 738, 1 450, 1 125, 960/cm.

d) 3-Methyl-2-oxo-non-5-inyl-phosphonsäure-dimethylester

Zu einer Lösung von 15,8 g Natrium in 340 ml Äthylalkohol tropft man bei 20 °C 120 g Methylmalonsäurediäthylester. Nach 30 Minuten tropft man 135 g 1-Brom-2-hexin (hergestellt aus Hex-2-in-1-ol mit Phosphortribromid in Pyridin) zu und erhitzt 16 Stunden unter Rückfluß. Anschließend filtriert man, wäscht den Rückstand mit Methylenchlorid und engt im Vakuum ein. Den Rückstand löst man in 500 ml Methylenchlorid, schüttelt zweimal mit je 50 ml Wasser, trocknet über Magnesiumsulfat und engt im Vakuum ein. Den Rückstand destilliert man im Vakuum bei 14 Torr und 148-152 °C. Man erhält als

Destillat 155 g des alkylierten Methylmalonsäureesters, den man in 1 200 ml Dimethylsulfoxid und 12 ml Wasser mit 52 g Lithiumchlorid 4,5 Stunden unter Rückfluß erhitzt. Anschließend gießt man auf 5 l Eiswasser, extrahiert mit Äther, schüttelt den Extrakt mit Wasser, trocknet über Magnesiumsulfat und engt im Vakuum ein. Die Destillation des Rückstandes liefert bei 94-96 °C und 14 Torr 95 g 2-Methyl-oct-4-insäureäthylester als farblose Flüssigkeit.

Zu einer Lösung von 176 g Methanphosphonsäuredimethylester in 2 l Tetrahydrofuran tropft man 640 ml einer 1,5 M Butyllithiumlösung in Hexan bei — 70 °C. Nach 15 Minuten fügt man langsam eine Lösung von 90 g 2-Methyl-oct-4-insäureäthylester in 300 ml Tetrahydrofuran zu. Man rührt 4 Stunden bei — 70 °C, neutralisiert mit Essigsäure und dampft im Vakuum ein. Man versetzt den Rückstand mit 200 ml Wasser, extrahiert dreimal mit je 500 ml Methylenchlorid, schüttelt den Extrakt mit 100 ml Wasser, trocknet über Magnesiumsulfat und engt in Vakuum ein. Die Destillation des Rückstandes liefert bei 0,35 Torr und 126-128 °C 80 g der Titelverbindung als farblose Flüssigkeit.

## Beispiel 4

(5E)-13,14-Didehydro-16,16-dimethyl-18,18,19,19-tetradehydro-6a-carba-prostaglandin-$I_2$

In Analogie zu Beispiel 1 erhält man aus 1,5 g (1R,5S,6R,7R)-7-(Tetrahydropyran-2-yloxy)-6-[(3S)-2-brom-4,4-dimethyl-3-(tetrahydropyran-2-yloxy)-oct-1-en-6-inyl]-bicyclo[3.3.0]octan-3-on 610 mg (5E)-13,14-Didehydro-16,16-dimethyl-18,18,19,19-tetradehydro-6a-carba-prostaglandin-$I_2$-11,15-bis-(tetrahydropyran-2-yläther) als farbloses Öl.

IR : 3 500 (breit), 2 944, 2 862, 2 222, 1 708/cm.

Nach Abspaltung der Schutzgruppen gemäß Beispiel 1 erhält man 290 mg der Titelverbindung als farbloses Öl.

IR : 3 600, 3 400 (breit), 2 930, 2 862, 1 708, 1 600/cm.

a) (1R,5S,6R,7R)-3,3-Äthylendioxy-7-benzoyloxy-6-(2-brom-4,4-dimethyl-3-oxo-oct-1-en-6-inyl)-bicyclo[3.3.0]octan

In Analogie zu Beispiel 1a) erhält man aus 6,25 g 3,3-Dimethyl-2-oxo-hept-5-in-phosphonsäuredimethylester 3,7 g N-Bromsuccinimid und 5,6 g (1R,5S,6R,7R)-3,3-Äthylendioxy-7-benzoyloxy-6-formyl-bicyclo[3.3.0]octan 4,7 g des ungesättigten Ketons als farbloses Öl.

IR : 2 940, 2 878, 1 710, 1 688, 1 602, 1 594, 1 448, 1 270, 944/cm.

b) (1R,5S,6R,7R)-7-Hydroxy-6-[(3S)-2-brom-4,4-dimethyl-3-hydroxy-oct-1-en-6-inyl]-bicyclo[3.3.0]octan-3-on

In Analogie zu Beispiel 1b) erhält man aus 4 g des nach Beispiel 4a) hergestellten Ketons nach Reduktion mit Natriumborhydrid, Verseifung mit Kaliumcarbonat und anschließender Ketalspaltung 1,40 g der Titelverbindung (15α-Hydroxy) als farbloses Öl.

IR : 3 600, 3 410 (breit), 2 958, 2 865, 1 738, 1 600/cm.

c) (1R,5S,6R,7R)-7-(Tetrahydropyran-2-yloxy)-6-[(3S)-2-brom-4,4-dimethyl-3-(tetrahydropyran-2-yloxy)-oct-1-en-6-inyl]-bicyclo[3.3.0]octan-3-on

In Analogie zu Beispiel 1c) erhält man aus 1,2 g des nach Beispiel 4b) hergestellten Diols mit Dihydropyran 1,6 g der Titelverbindung als Öl.

IR : 2 942, 2 870, 1 738, 1 450, 1 132, 960/cm.

## Beispiel 5

(5E)-13,14-Didehydro-18,18,19,19-tetradehydro-16,16,20-trimethyl-6a-carba-prostaglandin-$I_2$

In Analogie zu Beispiel 1 erhält man aus 1 g (1R,5S,6R,7R)-7-(Tetrahydropyran-2-yloxy)-6-[(3S)-2-brom-4,4-dimethyl-3-(tetrahydropyran-2-yloxy)-non-1-en-6-inyl]-bicyclo[3.3.0]octan-3-on 400 mg (5E)-13,14-Didehydro-18,18,19,19-tetradehydro-16,16,20-trimethyl-6a-carba-prostaglandin-$I_2$-11,15-bis-(tetrahydropyranyläther) als farbloses Öl.

IR : 3 510 (breit), 2 940, 2 858, 2 220, 1 708/cm.

Nach Abspaltung der Schutzgruppen gemäß Beispiel 1 erhält man 410 mg der Titelverbindung als farbloses Öl.

IR : 3 600, 3 340 (breit), 2 940, 2 832, 2 220, 1 708, 1 600/cm.

Das Ausgangsmaterial für die obige Titelverbindung wird wie folgt hergestellt :

a) (1R,5S,6R,7R)-3,3-Äthylendioxy-7-benzoyloxy-6-(2-brom-4,4-dimethyl-3-oxo-non-1-en-6-inyl)-bicyclo[3.3.0]octan

In Analogie zu Beispiel 1a) erhält man aus 12,6 g 3,3-Dimethyl-2-oxo-oct-5-inyl-phosphonsäuredimethylester 7,4 g N-Bromsuccinimid und 11,2 g (1R,5S,6R,7R)-3,3-Äthylendioxy-7-benzoyloxy-6-formylbicyclo[3.3.0]octan 8,7 g des ungesättigten Ketons als farbloses Öl.

IR : 2 946, 2 880, 1 712, 1 687, 1 601, 1 594, 1 272, 948/cm.

b) (1R,5S,6R,7R)-7-Hydroxy-6-[(3S)-2-brom-4,4-dimethyl-3-hydroxy-non-1-en-6-inyl]-bicyclo[3.3.0]-octan-3-on

In Analogie zu Beispiel 1b) erhält man aus 5 g des nach Beispiel 5a) hergestellten Ketons nach Reduktion mit Natriumborhydrid, Verseifung mit Kaliumcarbonat und anschließender Ketalspaltung 1,80 g der Titelverbindung (15α-Hydroxy) als farbloses Öl.

IR : 3 600, 3 404 (breit), 2 958, 2 864, 1 738, 1 601/cm.

c) (1R,5S,6R,7R)-7-(Tetrahydropyran-2-yloxy)-6-[(3S)-2-brom-4,4-dimethyl-3-(tetrahydropyran-2-yloxy)-non-1-en-6-inyl]-bicyclo[3.3.0]octan-3-on

In Analogie zu Beispiel 1c) erhält man aus 1,5 g des nach Beispiel 5b) hergestellten Diols 2,20 g der Titelverbindung als farbloses Öl.

IR : 2 942, 2 878, 1 738, 1 125, 968/cm.

## Beispiel 6

(5E)-13,14-Didehydro-18,18,19,19-tetradehydro-6a-carbaprostaglandin-$I_2$

In Analogie zu Beispiel 1 erhält man aus 400 mg (1R,5S,6R,7R)-7-(Tetrahydropyran-2-yloxy)-6-[(3S)-2-brom-3-(tetrahydropyran-2-yloxy)-oct-1-en-6-inyl]-bicyclo[3.3.0]octan-3-on 130 mg (5E)-13,14-Didehydro-18,18,19,19-tetradehydro-6a-carba-prostaglandin-$I_2$-11,15-bis-(tetrahydropyranyläther) als farbloses Öl.

IR : 3 500 (breit), 2 948, 2 862, 2 226, 1 708/cm.

Nach Abspaltung der Schutzgruppen gemäß Beispiel 1 erhält man 62 mg der Titelverbindung als farbloses Öl.

IR : 3 610, 3 350 (breit), 2 930, 2 862, 2 226, 1 709, 1 600/cm.

Das Ausgangsmaterial für die obige Titelverbindung wird wie folgt hergestellt :

a) (1R,5S,6R,7R)-3,3-Äthylendioxy-7-benzoyloxy-6-(2-brom-3-oxo-oct-1-en-6-inyl)-bicyclo[3.3.0]-octan

In Analogie zu Beispiel 1a) erhält man aus 5,8 g 2-Oxo-hept-5-inyl-phosphonsäure-dimethylester 3,7 g N-Bromsuccinimid und 5,6 g (1R,5S,6R,7R)-3,3-Äthylendioxy-7-benzoyloxy-6-formyl-bicyclo-[3.3.0]octan 4,7 g des ungesättigten Ketons als farbloses Öl.

IR : 2 932, 2 880, 1 712, 1 688, 1 600, 1 592, 1 272, 948/cm.

b) (1R,5S,6R,7R)-7-Hydroxy-6-[(3S)-2-brom-3-hydroxy-oct-1-en-6-inyl]-bicyclo[3.3.0]octan-3-on

In Analogie zu Beispiel 1b) erhält man aus 4 g des nach Beispiel 6a) hergestellten Ketons nach Reduktion mit Natriumborhydrid, Verseifung mit Kaliumcarbonat und anschließender Ketalspaltung 1,35 g der Titelverbindung als farbloses Öl.

IR : 3 600, 3 410 (breit), 2 962, 2 866, 1 740, 1 601/cm.

c) (1R,5S,6R,7R)-7-(Tetrahydropyran-2-yloxy)-6-[(3S)-2-brom-3-(tetrahydropyran-2-yloxy)-oct-1-en-6-inyl]-bicyclo [3.3.0]octan-3-on

In Analogie zu Beispiel 1c) erhält man aus 1,20 g des nach Beispiel 6b) hergestellten Diols mit Dihydropyran 1,61 g der Titelverbindung als farbloses Öl.

IR : 2 945, 2 882, 1 739, 1 125, 968/cm.

## Beispiel 7

(5E)-(16RS)-13,14-Didehydro-16-methyl-18,18,19,19-tetradehydro-6a-carba-prostaglandin-$I_2$-tris(hydroxymethyl)-aminomethansalz

Zu einer Lösung von 358 mg (5E)-(16RS)-13,14-Didehydro-16-methyl-18,18,19,19-tetradehydro-6a-carba-prostaglandin-$I_2$ in 60 ml Acetonitril gibt man bei 65 °C eine Lösung von 121 mg Tris-(hydroxymethyl)-aminomethan in 0,4 ml Wasser. Unter Rühren läßt man abkühlen, nach 16 Stunden dekantiert man vom Lösungsmittel und trocknet den Rückstand bei 25 °C und 0,1 Torr. Man erhält 310 mg der

Titelverbindung als wachsartige Masse.

**Patentansprüche**

1. Carbacyclinderivate der allgemeinen Formel I

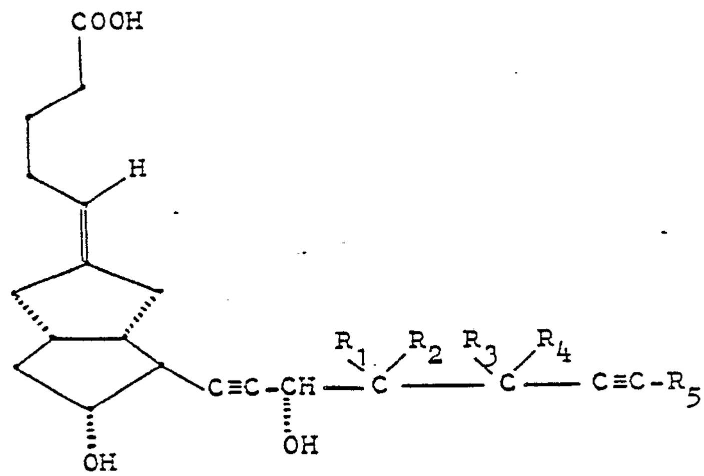

(I)

worin

$R_1$, $R_2$, $R_3$, $R_4$ ein Wasserstoffatom oder eine Alkylgruppe mit 1-2 C-Atomen,

$R_5$ eine Alkylgruppe mit 1-2 C-Atomen bedeuten, sowie deren Salze mit physiologisch verträglichen Basen.

2. (5E)-(16RS)-20-Äthyl-13,14-didehydro-16-methyl-18,18,19,19-tetradehydro-6a-carba-prostaglandin-$I_2$.

3. Verfahren zur Herstellung der Carbacyclinderivate der allgemeinen Formel I, dadurch gekennzeichnet, daß man eine Verbindung der allgemeinen Formel II

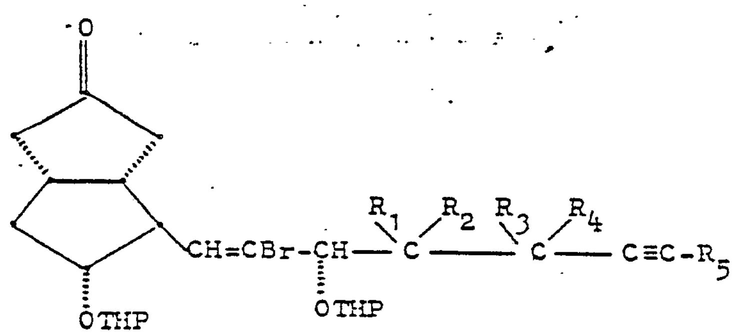

(II)

worin $R_1$, $R_2$, $R_3$, $R_4$, $R_5$ die oben angegebenen Bedeutungen aufweisen und THP den Tetrahydropyranylrest bedeutet mit einem Wittig-Reagenz der Formel III

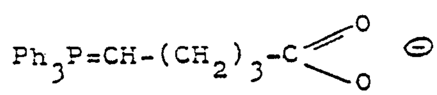

(III)

worin Ph eine Phenylgruppe bedeutet, unter gleichzeitiger HBr-Abspaltung umsetzt und anschließend Isomere trennt, Schutzgruppen abspaltet und gegebenenfalls die Carboxylgruppe mit einer physiologisch verträglichen Base in ein Salz überführt.

4. Arzneimittel, bestehend aus einer oder mehreren Verbindungen der Ansprüche 1 und 2 und üblichen Hilfs- und Trägerstoffen.

5. (5E)-(16RS)-13,14-Didehydro-16,20-dimethyl-18,18,19,19-tetradehydro-6a-carba-prostaglandin-$I_2$.

**Claims**

1. Carbacyclin derivatives of the general formula I

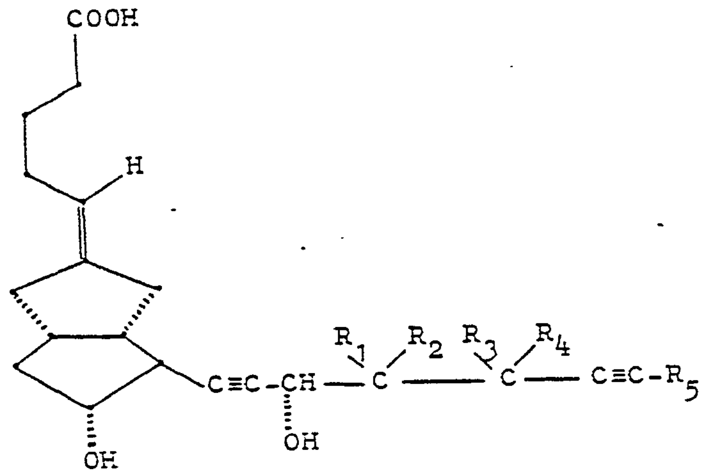

(I)

wherein

$R_1$, $R_2$, $R_3$ and $R_4$ are hydrogen or alkyl of 1-2 C-atoms.

$R_5$ is alkyl of 1-2 C-atoms, as well as their salts with pharmaceutically acceptable bases.

2. (5E)-(16RS)-20-Ethyl-13,14-didehydro-16-methyl-18,18,19,19-tetradehydro-6a-carbaprostaglandin-$I_2$.

3. Process for the preparation of carbacyclin derivatives of general formula I, characterised in that a compound of general formula II

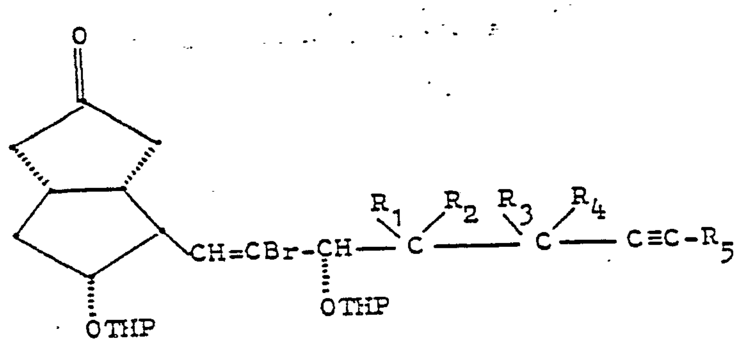

(II)

wherein $R_1$, $R_2$, $R_3$, $R_4$ and $R_5$ have the meanings given above and THP is a tetrahydropyranyl group, is reacted with a Wittig reagent of the formula III

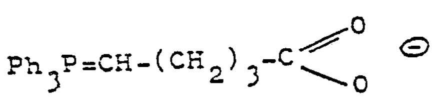

(III)

wherein Ph is a phenyl group, with simultaneous HBr cleavage followed by separation of the isomers, removal of the protecting group and optionally converting the carboxyl group to a salt with a physiologically acceptable base.

4. Pharmaceutical composition containing one or more compounds of claims 1 and 2 and conventional auxiliary and carrier materials.

5. (5E)-(16RS)-13,14-Didehydro-16,20-dimethyl-18,18,19,19-tetradehydro-6a-carbaprostaglandin-$I_2$.

**Revendications**

1. Carbacyclines répondant à la formule générale I :

(Voir formule p. 13)

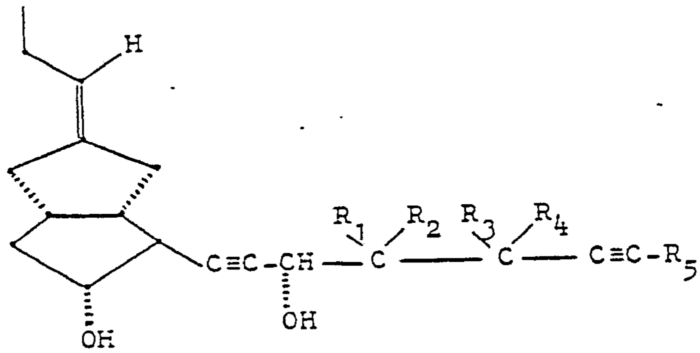

(I)

dans laquelle

$R_1$, $R_2$, $R_3$ et $R_4$ représentent chacun un atome d'hydrogène ou un radical alkyle contenant 1 ou 2 atomes de carbone,

et

$R_5$ représente un radical alkyle contenant 1 ou 2 atomes de carbone, ainsi que les sels qu'elles forment avec des bases acceptables du point de vue physiologique.

2. Ethyl-20 didéhydro-13,14 méthyl-16 tétradéhydro-18,18,19,19 carba-6a prostaglandine $I_2$ (5E)-(16RS).

3. Procédé de préparation des carbacyclines de formule générale I, procédé caractérisé en ce qu'on fait réagir, avec enlèvement simultané d'HBR, un composé répondant à la formule générale II :

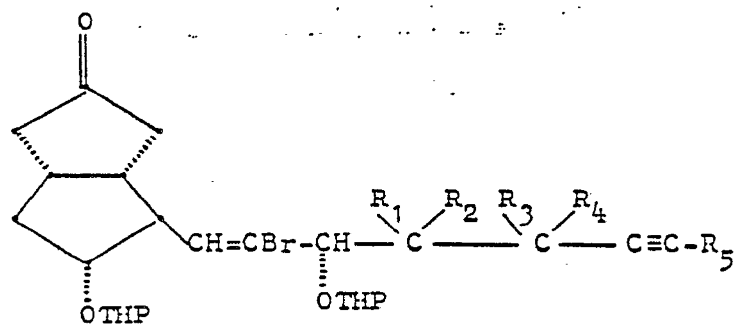

(II)

dans laquelle $R_1$, $R_2$, $R_3$, $R_4$ et $R_5$ ont les significations précédemment données et THP représente le radical tétrahydropyrannyle, avec un réactif de Wittig répondant à la formule III :

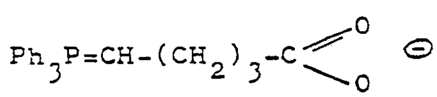

(III)

dans laquelle Ph représente un radical phényle, puis on sépare les isomères, on élimine les radicaux protecteurs et, éventuellement, on transforme le radical carboxy en un sel avec une base acceptable du point de vue physiologique.

4. Médicament constitué d'un ou de plusieurs composés selon l'une des revendications 1 et 2 ainsi que d'adjuvants et d'excipients usuels.

5. Didéhydro-13,14 diméthyl-16,20 tétradéhydro-18,18,19,19 carba-6a prostaglandine $I_2$ (5E)-(16RS).